## Europäisches Patentamt

## European Patent Office

## Office européen des brevets

(19)

(11) Veröffentlichungsnummer: **0 062 227**
**A1**

(12) **EUROPÄISCHE PATENTANMELDUNG**

(21) Anmeldenummer: **82102389.2**

(22) Anmeldetag: **23.03.82**

(51) Int. Cl.³: **G 01 N 33/96**

(30) Priorität: **28.03.81 DE 3112334**

(43) Veröffentlichungstag der Anmeldung: **13.10.82**
**Patentblatt 82/41**

(84) Benannte Vertragsstaaten: **AT BE CH DE FR GB IT LI LU NL SE**

(71) Anmelder: **BEHRINGWERKE Aktiengesellschaft, Postfach 1140, D-3550 Marburg 1 (DE)**

(72) Erfinder: **Becker, Wolfgang, Dr., Quotshäuserweg 20, D-3564 Steffenberg (DE)**
Erfinder: **Oeding, Volker, Dr., Zur Klause 7, D-3550 Marburg/Lahn (DE)**
Erfinder: **Pauly, Hans Erwin, Dr., Finkenstrasse 1, D-3563 Dautphetal (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) Künstliche Kontroll- und Standardseren, Verfahren zu ihrer Herstellung und ihre Verwendung.

(57) Es werden kovalente Verbindungen tierischer Antikörper mit menschlichen Immunglobulinen, ein Verfahren zu ihrer Herstellung sowie ihre Verwendung als künstliche Kontroll- und Standardseren in immunologischen Nachweis- und Bestimmungsverfahren beschrieben.

EP 0 062 227 A1

ACTORUM AG

- 1 -

BEHRINGWERKE AKTIENGESELLSCHAFT    Hoe 81/B 007
Dr.Ha/Bl.

Künstliche Kontroll- und Standardseren, Verfahren zu
ihrer Herstellung und ihre Verwendung

Die Erfindung betrifft künstliche positive Kontroll- und
Standardseren, ein Verfahren zu ihrer Herstellung sowie
ihre Verwendung in immunologischen Nachweisverfahren.

Übliche immunologische Verfahren zur Diagnose von Erkrankungen, die
mit einer Bildung von spezifischen Antikörpern gegen einen
Krankheitsauslöser, wie Viren, Bakterien, Allergene,
einhergehen, beruhen auf der Fähigkeit dieser Antikörper
zur Komplexbildung mit antigenen Strukturen des Auslösers.
Bei einigen dieser Verfahren wird eine auf den Gehalt an spezifischen Antikörpern zu prüfende Probe mit den antigenen
Strukturen des Krankheitsauslösers in Kontakt gebracht,
wobei diese antigenen Strukturen an geeignete Trägermaterialien befestigt sind. In der Probe enthaltene spezifische Antikörper werden als Immunkomplex an die auf dem
Trägermaterial fixierten antigenen Strukturen des Krankheitsauslösers gebunden und nachgewiesen. Dazu können in
Tieren gewonnene Nachweisantikörper, die zur Komplexbildung mit dem spezifischen Antikörper der Probe befähigt
sind, verwendet werden. Der Nachweisantikörper trägt in der
Regel eine Markierung, welche die Menge des gebundenen
spezifischen Antikörpers meßtechnisch erfaßbar macht.
Gebräuchliche Markierungen sind: Radioaktive Isotope,
Enzyme, fluoreszierende, phosphorezierende oder lumineszierende Substanzen, Substanzen mit stabilen, ungepaarten
Elektroden, Erythrozyten, Latexpartikel.

- 2 -

Für diese immunologischen Verfahren benötigt man Kontroll- und Standardseren, welche die nachzuweisenden Antikörper in definierter Menge enthalten. Ein derartiges positives Kontrollserum erlaubt es, die Brauchbarkeit der im Test verwendeten Reagenzien zu prüfen. Mit Hilfe von Standardseren sind quantitative Bestimmungen möglich.

Der derzeitige Stand der Technik der Herstellung von positiven Kontroll- oder Standardseren besteht darin, Patienten, deren Krankheit durch definierte Auslöser verursacht ist, Blut zu entnehmen, das Serum zu gewinnen und durch Mischen von Seren verschiedener Patienten das Kontroll- oder Standardserum auf einen bestimmten Gehalt an spezifischen, gegen den Krankheitsauslöser gerichteten Antikörper einzustellen.

Die Nachteile dieser Methode liegen darin, daß eine genügende Anzahl von Personen, deren Blut diese Antikörper enthält, verfügbar sein muß. Weiterhin sprechen oft medizinische Gründe gegen eine Blutentnahme bei solchen Personen, beispielsweise bei Kindern. Darüber hinaus ist es wichtig, zur Entnahme einen geeigneten Zeitpunkt im Krankheitsverlauf zu wählen, um einen bestimmten Typ an spezifischen Antikörpern zu erhalten; beispielsweise werden in der Frühphase einer Infektion vorwiegend Antikörper vom IgM-Typ gebildet.

Ziel der Erfindung war die Herstellung eines künstlichen, positiven Kontroll- oder Standardserums, wobei die oben angeführten Nachteile vermieden werden sollten.

Dieses Ziel wurde dadurch erreicht, daß mittels chemischer Verknüpfung ein Konjugat aus zwei Komponenten, A und B, hergestellt wurde, wobei die Komponente A dem Konjugat die Fähigkeit zur Komplexbildung mit den antigenen Strukturen des Krankheitsauslösers verleiht und die Komponente B die Eigenschaften beiträgt, die zur Komplexbildung mit dem markierten Nachweisantikörper erforderlich sind.

Zweckmäßigerweise ist die Komponente A ein Antikörper, der in üblicher Weise durch Immunisieren von Tieren mit dem Krankheitsauslöser gewonnen wird. Beispiele für die Komponente B sind alle im menschlichen Blut enthaltenen Klassen von Immunglobulin, die rein und in großer Menge nach bekannten Verfahren aus jedem menschlichen Serum isoliert werden können, sowie Immunglobulin-Fragmente, die zur Komplexbildung mit markierten Nachweisantikörpern fähig sind. Aus diesen Komponenten hergestellte Konjugate sind neu.

Zur Herstellung dieser Konjugate können alle dem Fachmann geläufigen Proteinverknüpfungsmethoden verwendet werden, insbesondere mittels homo- oder heterobifunktioneller Reagenzien, wie beispielsweise Glutaraldehyd oder N-Succinimidyl-3-(2-pyridyldithio)propionat. Besonders geeignet sind solche Methoden, bei denen die sterisch anspruchsvolle Komplexbildung sowohl des tierischen Antikörpers (Komponente A) mit den antigenen Strukturen des Krankheitsauslösers als auch die des Nachweisantikörpers mit dem menschlichen Immunglobulin oder dessen Fragmenten in geringstmöglicher Weise behindert wird (Komponente B).

Für die Herstellung von IgM-positiven Kontrollseren hat sich besonders die Verknüpfung über den Kohlenhydratanteil nach vorhergehender Modifizierung mit Methylacetimidat als vorteilhaft erwiesen (Beispiel 1).

- 4 -

Die für die A-Anteile der Konjugate benötigten tierischen Antikörper gegen den Krankheitsauslöser sind in den Immunseren von Kaninchen, Hammel, Ziege oder anderen Tieren verfügbar. Zur Konjugation kann abhängig von der verwendeten Verknüpfungsmethode eine immunadsorptiv oder auf andere Weise angereicherte Antikörperfraktion, die gesamte Gammaglobulinfraktion oder das Vollserum eingesetzt werden.

Die als B-Anteile bevorzugten menschlichen Immunglobuline können Immunglobuline der Klassen M, G, A, D und E als immunadsorptiv oder nach anderen an sich bekannten Verfahren gereinigte Fraktion, als Gammaglobulinfraktion oder als Vollserum von normalen menschlichen Blutspendern eingesetzt werden. Weiterhin sind Fragmente der Immunglobuline geeignet, beispielsweise H- oder L-Ketten, F(ab)-, Fc oder Fd-Bruchstücke, die sich nach literaturbekannten Verfahren herstellen lassen.

Zur Herstellung eines kübstlichen positiven Kontroll- oder Standardserums wird das vorstehend beschriebene Konjugat einer geeigneten Matrix zugesetzt. Diese kann humanes Normalserum sein; tierische Seren oder künstliche Matrices erscheinen jedoch in gleicher Weise verwendbar.

Das vorstehend beschriebene Verfahren wird anhand der folgenden Beispiele näher erläutert.

Beispiel 1   Kontrollserum für die Diagnose der Frühphase einer Syphilis-, Toxoplasmose- oder Chagas-Erkrankung (IgM-positives Kontrollserum)

Humanes IgM (5 mg/ml) wurde in 0,5 M Boratpuffer pH 9,5 mit einem $10^4$-fach molaren Überschuß an Methylacetimidat 2 Stunden bei Raumtemperatur zur Blockierung aller

- 5 -

intrinsischen, das heißt natürlicherweise vorhandenen
Aminogruppen inkubiert und dann ausgiebig
gegen Phosphat-gepufferte Kochsalzlösung pH 7,0 (PBS)
dialysiert. Das Acetamidino-IgM wurde mit einem 100-fach
molaren Überschuß an Natriumperjodat 1 Stunde bei Raumtemperatur inkubiert. Nach dem Stoppen der Reaktion mit
einem 500-fach molaren Überschuß Äthylenglykol (bezogen
auf Perjodat) wurde ausgiebig gegen PBS dialysiert. Die
Lösung wurde durch Zugabe von 2 M $K_2CO_3$-Lösung auf einen
pH-Wert von 9,5 eingestellt, aufgeteilt und

a) mit der Gammaglobulinfraktion eines gegen Syphilis-
   Erreger im Kaninchen gewonnenen Antiserums,
b) mit der Gammaglobulinfraktion eines gegen Toxoplasma
   gondii gewonnenen Antiserums vom Kaninchen beziehungsweise
c) mit der Gammaglobulinfraktion eines in Hammeln gewonnenen Antiserums gegen den Erreger der Chagas-Erkrankung

Jeweils 16 Stunden bei +4°C inkubiert. Die Endkonzentration in den Ansätzen betrug bezüglich IgM und der tierischen Gammaglobuline jeweils 2 mg/ml.

Anschließend wurde der pH-Wert der Ansätze durch Zugabe
von 1 N HCl auf pH 8,0 gebracht und die Lösung nach Zusatz von Natriumborhydrid (0,1 mg/ml des Ansatzes)
1 Stunde bei Raumtemperatur inkubiert, um die chemische
Bindung zwischen dem humanen IgM und den tierischen
Gammaglobulinen durch Reduktion in eine stabile Form zu
überführen.

Anschließend wurden die Produkte ausgiebig gegen PBS
dialysiert und normalem Humanserum in geeigneter Konzentration zugesetzt.

- 6 -

Beispiel 2  Anwendung der Konjugate im Immunfloureszenztest zum Nachweis der Toxoplasmose

Dazu werden abgetötete Toxoplasmen auf den Reaktionsfeldern von Objektträgern angetrocknet, so daß ca. 50
Organismen bei einer 400-fachen Vergößerung im Gesichtsfeld eines Mikroskops zu sehen sind. Die Reaktionsfelder
werden mit Lösungen aus einer geometrischen Verdünnungsreihe des in Beispiel 1b beschriebenen Konjugats bzw.
von menschlichen Serumproben überschichtet und 30 min
bei Raumtemperatur in einer feuchten Kammer inkubiert.
Durch mehrmaliges Waschen werden nichtgebundene Antikörper entfernt und die Reaktionsfelder mit einem /u-Kettenspezifischen Anti-Human-IgM (von der Ziege) beschichtet,
das mit FITC markiert ist. Nach erneutem Inkubieren und
Waschen werden die Präparate in gepufferter Glycerin-Lösung eingebettet und mit einem mit geeigneten Filterkombinationen ausgerüsteten Fluoreszenzmikroskop bei 400-
facher Vergößerung begutachtet. Als Kontrolle auf eventuelle Kreuzreaktivitäten wird in entsprechenden Verdünnungen das unbehandelte Kaninchenserum mitgeführt, aus dem
die tierischen Antikörper für das Konjugat gewonnen
wurden.                           *) Fluorescein-Isothiocyanat

Das Vorliegen einer frischen Infektion, deren Merkmal
das Auftreten von gegen den Errger gerichteten IgM-Antikörpern ist, läßt sich an dem gelbgrün-fluoreszierenden
Saum der Toxoplasmen leicht erkennen. Diese Fluoreszenz
resultiert aus der Bindung des FITC-markierten Nachweisantikörpers an die Toxoplasmen-spezifischen IgM-Antikörper von frisch erkrankten Patienten. In gleicher Weise
ließ sich das in der künstlichen Kontrollprobe enthaltene Konjugat aus in Kaninchen gewonnenen Antikörpern gegen
Toxoplasmen und menschlichem IgM in diesem Test nachweisen. Der gefundene positive Titer betrug 1 : 320.

- 7 -

Beispiel 3   Herstellung von Konjugaten aus humanem IgA
und Kaninchen-Anti-Gliadin-Antikörpern

Humanes IgA (1 mg) und die Gammaglobulinfraktion eines Anti-Gliadin-Antiserums vom Kaninchen (2 mg) wurden in 1 ml PBS mit 1 uMol Glutaraldehyd 2 Stunden bei Raumtemperatur inkubiert.  Die Reaktion wurde beendet durch Zusatz eines gleichen Volumens von 50 mM Tris-HCl-Puffer pH 7,0.

Das Produkt wurde gegen PBS 16 Stunden bei + 4$^{\circ}$C dialysiert und normalem Humanserum zugesetzt.

Unter identischen Bedingungen wurde reines menschliches IgG sowie eine nach einer publizierten Vorschrift (J. Allergy Clin. Immunol. 63 (3), 139 (1979)) gewonnene angereicherte IgE-Präparation umgesetzt.

Beispiel 4   Anwendung der Konjugate im Enzymimmunoassay

In einem Enzymimmunoassay zum Nachweis von Antikörpern gegen Gliadin wird das zu prüfende Serum bzw. die positive künstliche Kontrollprobe in mit Gliadin beschichteten Polystyrolröhrchen 2 Stunden inkubiert. Dabei binden sich die spezifischen Antikörper an Gliadin und werden nach einem Waschschritt mit Peroxidase-markierten tierischen Antikörpern gegen die betreffende menschliche Immunglobulinklasse quantitativ nachgewiesen. Das Markerenzym Peroxidase vermag die Bildung eines Farbstoffs zu katalysieren; die nach 30 min erzeugte Farbe ist proportional zum Gehalt der Probe an gegen Gliadin gerichteten Antikörpern. In diesem Sandwich-Enzymimmunoassay zum Nachweis von Gliadin-spezifischen Antikörpern wurden die drei künstlich positiven Kontrollseren eingesetzt, deren Herstellung in Beispiel 3 beschrieben ist. Die durch die Farbstoff-bildende Reaktion des Markierungs-

- 8 -

enzyms erhaltenen Extinktionswerte·sind für unterschiedliche Verdünnungen der einzelnen Konjugate in der folgenden
Tabelle zusammengestellt.

| Verdünnung | Extinktion bei 492 nm | | Verdünnung | Extinktion bei 492 nm |
| | IgA-Nach-weis | IgG-Nach-·weis | | IgE-Nachweis |
| --- | --- | --- | --- | --- |
| 1 : 400 | 2.6 | 1.·54 | 1 : 10 | .435 |
| 1 : 800 | 2.24 | 1.35 | 1 : 20 | .34 |
| 1 : 1600 | 1.3 | 1.1 | 1 : 40 | .14 |
| 1 : 3200 | 0.92 | 0.74 | 1 : 80 | .082 |
| 1 : 6400 | 0.53 | 0.47 | 1 : 160 | .055 |
| 1 : 12800 | 0.29 | 0.3 | 1 : 320 | .031 |

Patentansprüche :

1.  Eine kovalente Verbindung eines tierischen Antikörpers mit einem menschlichen Immunglobulin oder
    Immunglobulin-Fragment..

2.  Eine Verbindung nach Anspruch 1, dadurch gekennzeichnet, daß der tierische Antikörper und das menschliche
    Immunglobulin oder Immunglobulin-Fragment mit Hilfe
    an sich bekannter proteinchemischer Verknüpfungsmethoden miteinander verbunden werden.

3.  Eine Verbindung nach Anspruch 1, dadurch gekennzeichnet, daß als menschliches Immunglobulin die Immunglobuline des Vollserums, der Gammaglobulinfraktion oder
    einer gereinigten Proteinfraktion in der Verbindung
    vorhanden sind.

4.  Eine Verbindung nach Anspruch 1, dadurch gekennzeichnet, daß als menschliches Immunglobulin ein Immunglobulin der Klasse M, G, A, E oder D oder ein Fragment
    von diesen in der Verbindung vorhanden ist.

5.  Verfahren zur Herstellung einer Verbindung aus einem
    tierischen Antikörper und einem menschlichen Immunglobulin oder Immunglobulin-Fragment, dadurch gekennzeichnet, daß ein tierischer Antikörper mit Hilfe
    eines an sich bekannten proteinchemischen Verknüpfungsverfahrens mit einem menschlichen Immunglobulin
    oder Immunglobulinfragment verbunden wird.

6.  Verwendung einer Verbindung nach einem der Ansprüche
    1 bis 4 in einem künstlichen Kontroll- oder Standard-
    serum.

Patentansprüche für Österreich

1. Ein Verfahren zur Herstellung einer Verbindung aus einem tierischen Antikörper und einem menschlichen Immunglobulin oder Immunglobulin-Fragment, dadurch gekennzeichnet, daß ein tierischer Antikörper mit Hilfe eines an sich bekannten proteinchemischen Verknüpfungsverfahrens mit einem menschlichen Immunglobulin oder Immunglobulinfragment verbunden wird.

2. Ein Verfahren nach Anspruch 1, dadurch gekennzeichnet, daß als menschliches Immunglobulin die Immunglobuline des Vollserums, der Gammaglobulinfraktion oder einer gereinigten Proteinfraktion mit einem tierischen Antikörper verbunden werden.

3. Ein Verfahren nach Anspruch 1, dadurch gekennzeichnet, daß als menschliches Immunglobulin ein Immunglobulin der Klasse M, G, A, E oder D oder ein Fragment von diesen mit einem tierischen Antikörper verbunden werden.

4. Die Verwendung einer nach einem der Ansprüche 1 bis 3 erhältlichen Verbindung in einem künstlichen Kontroll- oder Standardserum.

0062227

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US – A – 3 980 764 (R.J. ADAMS)<br>* Anspruch 6; Beispiele 1, 2 * | 1-5 |
| A | DE – A1 – 2 902 677 (TECHNICON INSTRUMENTS CO.) | |

**KLASSIFIKATION DER ANMELDUNG (Int. Cl.³)**

G 01 N 33/96

**RECHERCHIERTE SACHGEBIETE (Int. Cl.³)**

A 61 K 39/395
G 01 N 33/96

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 18-06-1982 | SCHWARTZ |

EPA form 1503.1  06.78